(19) Europäisches Patentamt / European Patent Office / Office européen des brevets

(11) **EP 4 738 269 A1**

(12) **EUROPEAN PATENT APPLICATION**

(43) Date of publication:
06.05.2026 Bulletin 2026/19

(21) Application number: 25210069.8

(22) Date of filing: 21.10.2025

(51) International Patent Classification (IPC):
*G06T 17/00* (2006.01)

(52) Cooperative Patent Classification (CPC):
**G06T 17/00; G06T 19/00;** G06T 2210/41;
G06T 2210/56

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 29.10.2024 IN 202421082881

(71) Applicant: **Tata Consultancy Services Limited**
**Mumbai, Maharashtra 400 021 (IN)**

(72) Inventors:
• **CHANDEL, Vivek**
**122004 Gurugram, Haryana (IN)**

• **BHATIA, Divya Manoharlal**
**560009 Bangalore, Karnataka (IN)**
• **KANAKATTE GURUMURTHY, Aparna**
**560066 Bangalore, Karnataka (IN)**
• **KHANDELWAL, Sundeep**
**201309 Noida, Uttar Pradesh (IN)**
• **GHOSE, Avik**
**700135 Kolkatta, West Bengal (IN)**
• **SINHA, Aniruddha**
**700091 Kolkata, West Bengal (IN)**

(74) Representative: **Goddar, Heinz J. et al**
**Boehmert & Boehmert**
**Anwaltspartnerschaft mbB**
**Pettenkoferstrasse 22**
**80336 München (DE)**

(54) **METHODS AND SYSTEMS FOR 3-D RECONSTRUCTION AND VISUALIZATION OF ANATOMY USING 2-D IMAGE SLICES**

(57) The disclosure relates generally to methods and systems for 3-D reconstruction and visualization of anatomy using 2-dimensional (2-D) image slices. Conventional techniques lack in modelling the internal structures of the anatomy where mesh models of the internal structures are not visible when outer surface is visualized. According to the present disclosure, the 3-D reconstruction and visualization of the anatomy using the 2-D image slices is done in three steps. In first step, the multi-directional image information of the anatomy is combined in the form of a point cloud and converted into a voxel volume. In the second step, a 3-D mesh surface is created for each layered component associated with the anatomy. In third step, the layered mesh surfaces of the reconstructed anatomy are cut and opened in multiple directions representing multiple components to represent artifacts such as covering portions and details internal to the outer wall of the anatomy.

3-D mesh surface for blood being visualized together with meshes of inner and outer walls, and the covering mesh to provide a detailed and structurally accurate view of the 3-D meshes of components of interest

FIG. 17

EP 4 738 269 A1

**Description**

CROSS-REFERENCE TO RELATED APPLICATIONS AND PRIORITY

The present application claims priority from Indian provisional application no. 202421082881, filed on October 29, 2024.

TECHNICAL FIELD

The disclosure herein generally relates to 3-dimensional (3-D) model generation, and, more particularly, to methods and systems for 3-D reconstruction and visualization of anatomy using 2-dimensional (2-D) image slices.

BACKGROUND

Reconstructing a 3-dimensional (3-D) model of an anatomy such as heart, lungs, kidney, and dental arch, of a living being such as humans and animals, plays a vital role for better analytics and diagnostics. Making use of a set of 2-dimensional (2-D) image slices of the anatomy that are acquired directly from imaging techniques such as Magnetic resonance imaging (MRI) and computed tomography (CT) imaging is one of most popular techniques for reconstructing the 3-dimensional (3-D) model of an anatomy.

Conventional techniques for reconstructing the 3-D model of the anatomy make use of the set of parallel 2-D image slices of the anatomy for generating a 3-D voxel volume and a 3-D mesh which is further used for reconstructing the 3-D model. But with limited number of 2-D image slices which are acquired from a single direction, the conventional techniques results in inaccurate and incomplete 3-D mesh generation and hence the 3-D model reconstructed may not be visualized with greater details of the anatomy. Further, the conventional techniques concern with creating the 3-D mesh representation of a voxel volume to provide external structure reflecting outer morphology of the Anatomy. The anatomy possesses greater details layered in the inner portion of the outer hull, which remain un-constructed for example, inner walls, presence of blood, etc.

Hence the conventional techniques lack in modelling the internal structures of the anatomy due to the reason that mesh models of the internal structures are not visible when the outer surface is visualized. It's imperative for a proper diagnostic activity involving detecting abnormalities in the internal structures (inner walls, blood, etc.) of the anatomy, or other congenital defects to not only look at the outer surface, but also be able to cut open the 3-D model from various directions and be able to visualize the inner details (structures) including inner walls, blood etc., together with the outer hull as 3-D mesh surfaces.

SUMMARY

Embodiments of the present disclosure present technological improvements as solutions to one or more of the above-mentioned technical problems recognized by the inventors in conventional systems.

In an aspect, a processor-implemented method for 3-D reconstruction and visualization of anatomy using 2-D image slices is provided. The method comprising: acquiring a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices; generating a point cloud associated with the set of 2-D image slices acquired at each of the one or more time-instances, wherein the point cloud comprises a plurality of points representing a plurality of pixels associated with the set of 2-D image slices of the anatomy acquired at each time-instance; creating a coherent voxel volume associated with the set of 2-D image slices acquired at each time-instance, from the point cloud associated with the set of 2-D image slices, using a 3-dimensional (3-D) interpolation technique; creating a 3-D mesh surface for each layered component of one or more layered components associated with the anatomy, using (i) the coherent voxel volume associated with the set of 2-D image slices acquired at each time-instance, and (ii) an iso-surface tracing technique; and creating a structurally complete sliced view of the 3-D mesh surface of the anatomy, from the 3-D mesh surface of each layered component of the one or more layered components associated with the anatomy created for each time-instance, using a mesh manipulation and rendering technique.

In another aspect, a system for 3-D reconstruction and visualization of anatomy using 2-D image slices is provided. The system includes: a memory storing instructions; one or more Input/Output (I/O) interfaces; and one or more hardware processors coupled to the memory via the one or more I/O interfaces, wherein the one or more hardware processors are configured by the instructions to: acquire a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices; generate a point cloud associated with the set of 2-D image slices acquired at each of the one or more time-instances, wherein the point cloud comprises a plurality of points representing a plurality of pixels associated with the set of 2-D image slices of the anatomy acquired at each time-instance; create a coherent voxel volume associated with the set of 2-D image slices acquired at each time-instance, from the point cloud associated with the set of 2-D image slices, using a 3-dimensional (3-D) interpolation technique; create a 3-D mesh surface for each layered component of one or more layered components associated with the anatomy, using (i) the coherent voxel volume associated with the set of 2-D image slices acquired at each time-instance, and (ii) an iso-surface tracing technique; and create a structurally complete sliced view of the 3-D mesh surface of the anatomy, from the 3-D mesh surface of each layered component of the one or more layered components associated with the anatomy created for each time-instance, using a mesh manipulation and rendering technique.

In yet another aspect, there are provided one or more non-transitory machine-readable information storage mediums comprising one or more instructions which when executed by one or more hardware processors cause: acquiring a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices; generating a point cloud associated with the set of 2-D image slices acquired at each of the one or more time-instances, wherein the point cloud comprises a plurality of points representing a plurality of pixels associated with the set of 2-D image slices of the anatomy acquired at each time-instance; creating a coherent voxel volume associated with the set of 2-D image slices acquired at each time-instance, from the point cloud associated with the set of 2-D image slices, using a 3-dimensional (3-D) interpolation technique; creating a 3-D mesh surface for each layered component of one or more layered components associated with the anatomy, using (i) the coherent voxel volume associated with the set of 2-D image slices acquired at each time-instance, and (ii) an iso-surface tracing technique; and creating a structurally complete sliced view of the 3-D mesh surface of the anatomy, from the 3-D mesh surface of each layered component of the one or more layered components associated with the anatomy created for each time-instance, using a mesh manipulation and rendering technique.

In an embodiment, the set of 2-D image slices of the anatomy is acquired in parallel from one or more predefined directions at each time-instance and at a predefined inter-slice distance in each of the one or more predefined directions.

In an embodiment, each of the one or more multi-directional 2-D image slices comprises the plurality of pixels and each of the plurality of pixels comprises (i) a 3-dimensional (3-D) location with reference to a fixed coordinate system, and (ii) one or more brightness values.

In an embodiment, the coherent voxel volume associated with the set of 2-D image slices acquired at each time-instance, is created by: defining a bounding box in a 3-D space, using the point cloud associated with the set of 2-D image slices acquired at each time-instance; creating a voxel space based on the bounding box associated with the set of 2-D image slices acquired at each time-instance using the 3-D interpolation technique, wherein the voxel space comprises a plurality of voxels and wherein each of the plurality of voxels is defined as a cube in the 3-D space with a side of a predefined length; and creating the coherent voxel volume associated with the set of 2-D image slices acquired at each time-instance, using the voxel space associated with the set of 2-D image slices acquired at each time-instance.

In an embodiment, the 3-D mesh surface for each layered component of the one or more layered components associated with the anatomy, is created by: identifying the one or more layered components associated with the anatomy, from the coherent voxel volume, using a predefined set of annotated 2-D slice images of the anatomy; extracting a scattered set of locations in the 3-D space associated with each layered component of one or more layered components, from the coherent voxel volume associated with the set of 2-D image slices acquired at each time-instance; and creating the 3-D mesh surface for each of the one or more layered component based on the scattered set of locations associated with each layered component, using the iso-surface tracing technique.

In an embodiment, the structurally complete sliced view of the 3-D mesh surface of the anatomy, is created by: slicing the 3-D mesh surface associated with each layered component of the one or more layered components associated with the anatomy, along one or more directions defined by a plane in the 3-D space, using the mesh manipulation and rendering technique, to obtain a sliced view of the 3-D mesh surface associated with each layered component; extracting one or more covering portions within each layered component and between the one or more layered components associated with the anatomy, based on the sliced view of the 3-D mesh surface associated with each layered component; and creating the meaningful sliced view of the 3-D mesh surface of the anatomy by rendering the one or more covering portions within each layered component and between the one or more layered components with a covering mesh using the mesh manipulation and rendering technique.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is an exemplary block diagram of a system for 3-D reconstruction and visualization of anatomy using 2-D image slices, in accordance with some embodiments of the present disclosure.

FIG. 2 illustrates an exemplary flow diagram of a processor-implemented method for 3-D reconstruction and visualization of anatomy using 2-D image slices, using the system of FIG. 1, in accordance with some embodiments of the present disclosure.

FIG. 3 shows an exemplary point cloud in grey-scale representing pixels from MRI 2-D image slices captured from multiple directions for a heart, in accordance with some embodiments of the present disclosure.

FIG. 4 is a flowchart showing the steps for creating a coherent voxel volume associated with the set of 2-D image slices acquired at each time-instance, in accordance with some embodiments of the present disclosure.

FIG. 5 shows an exemplary coherent voxel volume of heart in grey-scale created from the exemplary point cloud shown in FIG. 3, in accordance with some embodiments of the present disclosure.

FIG. 6 is a flowchart showing the steps for creating a 3-D mesh surface for each layered component of one or more layered components associated with the anatomy, in accordance with some embodiments of the present disclosure.

FIG. 7 shows an exemplary 3-D mesh surface of outer hull of the heart derived from the exemplary voxel volume shown in FIG. 5, in a plane across which a cut can be made to view internal structures, in accordance with some embodiments of the present disclosure.

FIG. 8 shows an exemplary mesh surface of the outer wall of 3-D mesh of heart cut along the plane depicted in FIG. 7, revealing the inside portion, in accordance with some embodiments of the present disclosure.

FIG. 9 shows an exemplary mesh surface of inner wall component of interest of heart cut along the plane depicted in FIG. 7, revealing significantly more details than cut view of just the outer wall depicted in FIG. 8, in accordance with some embodiments of the present disclosure.

FIG. 10 shows an exemplary mesh of blood component of interest of heart cut along the plane depicted in FIG. 7, revealing detailed placement of blood in different parts of the organ, in accordance with some embodiments of the present disclosure.

FIG. 11 shows an exemplary sliced meshes of both inner and outer walls of the heart cut along the plane as depicted FIG. 7 placed together with visible empty space between them, which is otherwise a solid area of heart muscle and fat in actuality, in accordance with some embodiments of the present disclosure.

FIG. 12 shows an exemplary 2-D slice contour derived by cutting the mesh of the outer wall depicted in FIG. 8, in accordance with some embodiments of the present disclosure.

FIG. 13 is a flowchart showing the steps for creating a structurally complete sliced view of the 3-D mesh surface of the anatomy, in accordance with some embodiments of the present disclosure.

FIG. 14 shows exemplary 2-D slice contours of both the outer and inner walls derived by cutting the outer wall and the inner wall meshes depicted in FIG. 8 and FIG. 9 respectively, in accordance with some embodiments of the present disclosure.

FIG. 15 shows an exemplary subtracted mesh representing one or more covering portions, in accordance with some embodiments of the present disclosure.

FIG. 16 shows an exemplary structurally complete sliced view where the 3-D meshes of both inner and outer walls of the heart are cut along the plane and placed together along with the covering portions depicting the solid wall, in accordance with some embodiments of the present disclosure.

FIG. 17 shows an exemplary structurally complete sliced view where the 3-D meshes of both inner and outer walls of the heart are cut along the plane and placed together along with the covering portions depicting the solid wall and the mesh surface representing the blood flow, in accordance with some embodiments of the present disclosure.

FIG. 18 shows an exemplary structurally complete sliced view with 3-D meshes of both inner and outer walls of the heart, and the 3-D mesh surface representing the blood flow cut along the plane of medically relevant 2-chamber short axis view, along with the covering portions (mesh lid) depicting the solid wall, in accordance with some embodiments of the present disclosure.

FIG. 19 shows an exemplary structurally complete sliced view with 3-D meshes of both inner and outer walls of the heart, and the 3-D mesh surface representing the blood flow cut along the plane of medically relevant 2-chamber long axis view, along with the covering portions (mesh lid) depicting the solid wall, in accordance with some embodiments of the present disclosure.

FIG. 20 shows an exemplary structurally complete sliced view with 3-D meshes of both inner and outer walls of the heart, and the 3-D mesh surface representing the blood flow cut along the plane of medically relevant 3-chamber view, along with the covering portions (mesh lid) depicting the solid wall, in accordance with some embodiments of the present disclosure.

FIG. 21 shows an exemplary structurally complete sliced view with 3-D meshes of both inner and outer walls of the heart, and the 3-D mesh surface representing the blood flow cut along the plane of medically relevant left-ventricle-outflow-tract view, along with the covering portions (mesh lid) depicting the solid wall, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

In the context of present disclosure, the words such as 'mesh', 'mesh surface', '3-D mesh', and '3-D mesh surface' are interchangeably used which mean the 3-D mesh of the anatomy and its surface. Further, the words such as 'anatomy', 'organ', and 'body part' are interchangeably used which mean the anatomy of the living being for which the 3-D reconstruction and visualization is to be created. Furthermore, the words such as 'parts', 'components' are used which mean the parts of the given anatomy.

Reconstructing a 3-dimensional (3-D) model of an anatomy such as heart, lungs, kidney, and dental arch, of a living being such as humans and animals, plays a vital role for better analytics and diagnostics. Creating a 3-D mesh surface of external structure (such as outer hull) for reconstruction of the 3-D model of an anatomy may be performed using multiple conventional techniques, but creation of internal details require separately demarcation of the internal components (internal structures such as inner walls, blood flow, tissues, and so on), which sometimes not very well defined even for a manual annotation (e.g. presence of blood in various parts of an organ).

In order to view the cut out internal details of the anatomy in its 3-D mesh form reconstructed from imaging output, it is imperative to include details which are internal to the outer surface. Cutting layered meshes also incorporates artifacts such as empty space between inner and outer structure of a solid muscle/flesh wall, which needs to be countered so that the final sliced view is meaningful. However, the conventional techniques lack in cutting of the mesh surface of reconstructed anatomy, the layered reconstruction of components of the anatomy is quite challenging.

The present disclosure solves the technical problems in the art with the methods and systems for 3-D reconstruction and visualization of anatomy using 2-D image slices. According to the present disclosure, the 3-D reconstruction and visualization of the anatomy using the 2-D image slices is done in three following steps as mentioned below. In the first step, the multi-directional image information of the anatomy is combined in the form of a point cloud and converted into a voxel volume. In literature, most of the conventional techniques concerned with the 3-D reconstruction for which the imaging techniques (such as MRI, CT) produce the 2-D slice images in same direction but in parallel manner, which are relatively straightforward to interpret and combine (creating the voxel volume using the parallel 2-D image slices can be performed by defining the voxel using corresponding pixels in two adjoining image slices and distance between the image slices). However, combining the multi-directional image slice information in the form of the point cloud and converting that to create the voxel volume is a non-trivial approach.

In the second step, a 3-D mesh surface is created for each layered component associated with the anatomy from the voxel volume using an iso-surface tracing technique. Tracing multiple surface contours in the domain of imaging along the voxel volume and creating different layered mesh surfaces for every value representing a component of interest, relevant to one or more parts of the anatomy, is non-trivial and has not been approached by the conventional techniques in this manner. In the third and last step, the layered mesh surfaces of the reconstructed anatomy are cut and opened in multiple medically relevant directions representing multiple components to represent artifacts such as covering portions (such as solid flesh/muscle), the details internal to the outer wall of the anatomy, and so on. Cutting and opening the layered mesh surfaces of the reconstructed anatomy is also non-trivial, since it requires presence of internal structure meshes in the first place (which is lacking as explained in the second step).

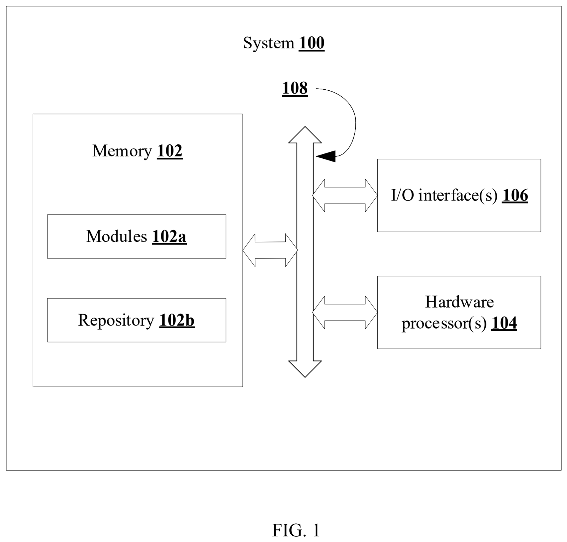

Referring now to the drawings, and more particularly to FIG. 1 through FIG. 21, where similar reference characters denote corresponding features consistently throughout the figures, there are shown preferred embodiments, and these embodiments are described in the context of the following exemplary system and/or method.

FIG. 1 is an exemplary block diagram of a system 100 for 3-D reconstruction and visualization of anatomy using 2-D image slices, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

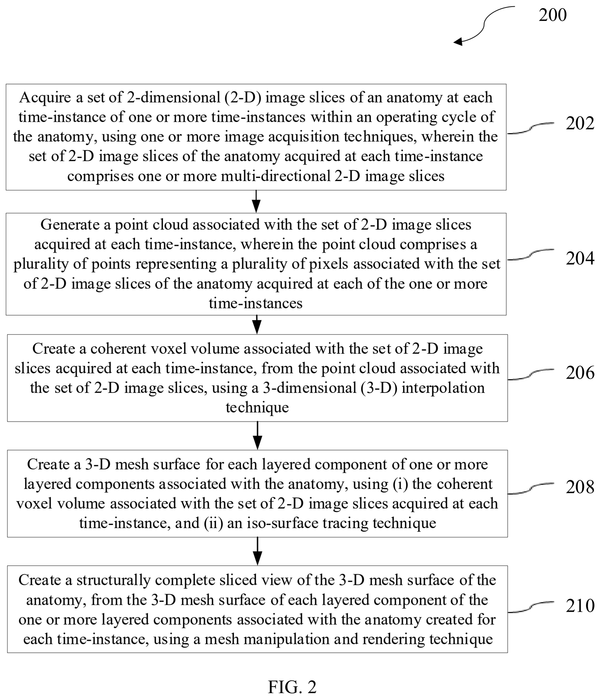

Referring to FIG. 2, components and functionalities of the system 100 are described in accordance with an example embodiment of the present disclosure. For example, FIG. 2 illustrates an exemplary flow diagram of a processor-implemented method 200 for 3-D reconstruction and visualization of anatomy using 2-D image slices, using the system of FIG. 1, in accordance with some embodiments of the present disclosure. Although the steps of the method 200 shown in FIG. 2 include process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method 200, the one or more hardware processors 104 of the system 100 are configured to acquire a set of 2-dimensional (2-D) image slices of the anatomy whose 3-D reconstruction and visualization are of interest. In an embodiment, the anatomy is a body part of any living being such as humans, animals, and so on. In an embodiment, the body part may include but are not limited to an internal organ such as heart, lungs, kidneys, brain, or any other part such as neck, hands, legs. The methods and systems of the present disclosure is concerned more of heart, lungs, kidneys, and brain of a human, however the scope of the present disclosure extends to other body parts and living being. In an embodiment, the set of 2-dimensional (2-D) image slices of the anatomy are acquired using one or more image acquisition techniques, such as magnetic resonance imaging (MRI), computed tomography (CT), and the like.

The set of 2-dimensional (2-D) image slices of the anatomy are acquired at each time-instance of one or more time-instances that are defined within an operating cycle of the anatomy. The operating cycle of the anatomy is defined as a repetitive minimum action of the anatomy to affect its intended function. For example, for heart the operating cycle spans one heartbeat (one systole-diastole cycle), for lungs it can be one respiratory cycle, and so on. For organs with no defined operation cycle like kidneys, liver etc., a time period can be selected by the specialist across which to visualize the organ function.

For example, the operating cycle of the anatomy refers to one systole-diastole cycle for heart (one heartbeat), one respiratory cycle for lungs, and so on. The anatomy that is not related to the operating cycle is considered with a predefined time period. For example, for organs such as brain, kidney, or liver, the time period can be selected as the operating cycle, that best visualizes the organ function. In another example, the operating cycle of the anatomy is considered during with a particular subject is performed an activity such as eating, reading, and resting.

Further, the set of 2-D image slices of the anatomy acquired at each time-instance includes one or more multi-directional 2-D image slices. Each multi-directional 2-D image slice of the one or more multi-directional 2-D image slices can be of same or different dimensions from each other along with a length and a width. There can be multiple parallel 2-D image slices at a predefined inter-slice distance in a given direction. So, the one or more multi-directional 2-D image slices of each set of 2-D image slices are acquired in parallel and from one or more predefined directions at each time-instance and at a predefined inter-slice distance within each of the one or more predefined directions. Thus, every operating cycle includes 2-D image slice captures at $N_c$ no. of time-instances, which can be uniformly spread throughout the operating cycle.

For example, if the operation cycle of the heart for the systole-diastole cycle is 1 second, number of the one or more time-instances is 30, the number of the one or more predefined directions is 5 and the predefined inter-slice distance is 1 millimeter, then for each of the 30 time instances of 1/30s duration each, the 2-D image slices are acquired in parallel with the distance between each image slice of 1 millimeter from each direction of the 5 directions.

Each multi-directional 2-D image slice of the one or more multi-directional 2-D image slices includes the plurality of pixels. Each pixel of the plurality of pixels at least includes two properties such as: (i) a 3-dimensional (3-D) location with reference to a fixed coordinate system, and (ii) one or more brightness values.

At step 204 of the method 200, the one or more hardware processors 104 of the system 100 are configured to generate a point cloud associated with the set of 2-D image slices acquired at each time-instance. Every pixel in every multi-directional 2-D image slice is assumed to be a point in the 3-D space. Thus, the point cloud includes a plurality of points representing the plurality of pixels associated with the set of 2-D image slices of the anatomy acquired at each time-instance. In simple terms, the point cloud associated with the set of 2-D image slices acquired at each time-instance is generated by combining the one or more multi-directional 2-D image slices present in the associated set of 2-D image slices.

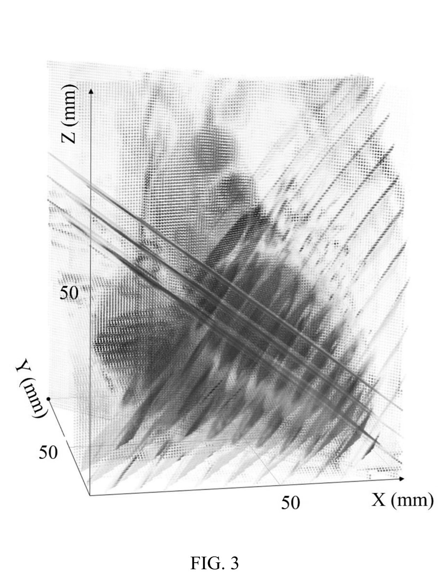

Thus, each point of the plurality of points present in the point cloud is represented with the 3-D location and holds a scalar value which represents the brightness value of the corresponding 3-D location in the associated multi-directional 2-D image slice of the one or more multi-directional 2-D image slices. The point cloud $P_i$ for the given one or more multi-directional 2-D image slices captured at time instance $t_i$ can be mathematically represented as $P_i = (P_{il}, P_{ib})$, where $P_{il}$ is the set of 3-D locations of all the points in the point cloud, and $P_{ib}$ is the set of brightness values of all the points in the point cloud, with points in the same order in both $P_{il}$ and $P_{ib}$. FIG. 3 shows an exemplary point in grey-scale representing pixels from MRI 2-D image slices captured from multiple directions for a heart, in accordance with some embodiments of the present disclosure.

At step 206 of the method 200, the one or more hardware processors 104 of the system 100 are configured to create a coherent voxel volume associated with the set of 2-D image slices acquired at each time-instance, from the point cloud of the associated set of 2-D image slices. A 3-dimensional (3-D) interpolation technique is employed to create the coherent voxel volume from the associated point cloud. Creating the voxel volume using parallel 2-D images slices can be performed by defining a voxel using corresponding pixels in two adjoining image slices and distance between the image slices using conventional techniques. However, the conventional lack in mixing information from 2-D image slices in multiple directions to create one coherent voxel volume.

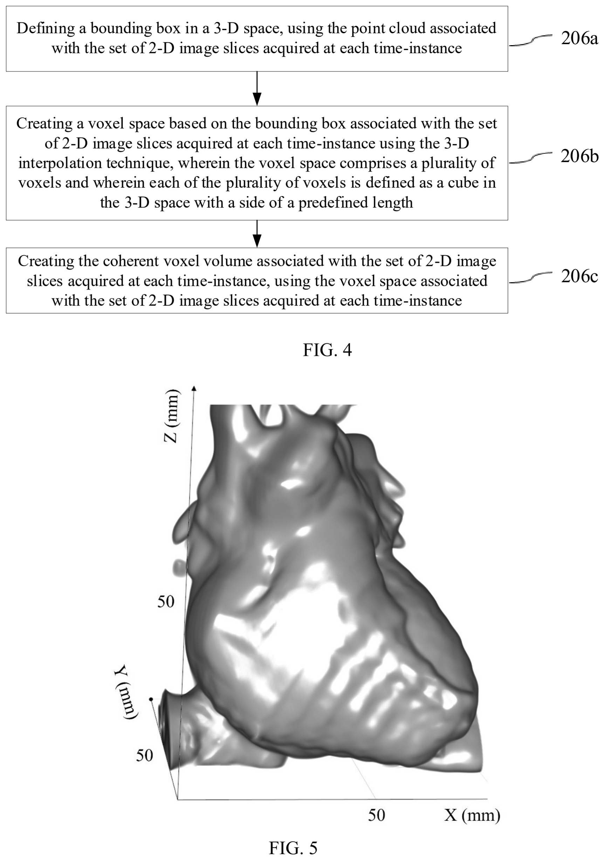

FIG. 4 is a flowchart showing the steps for creating a coherent voxel volume associated with the set of 2-D image slices acquired at each time-instance, in accordance with some embodiments of the present disclosure. As shown in FIG. 4, creating the coherent voxel volume associated with the set of 2-D image slices acquired at each time-instance is explained through steps 206a to 206c.

At step 206a, a bounding box is defined in a 3-D space, using the point cloud associated with the set of 2-D image slices acquired at the associated time-instance. Most specifically, the bounding box is defined in the shape of cuboid in the 3-D space, using the point cloud $P_i$ associated with the one or more multi-directional 2-D slices captured (of the set of 2-D image slices) at time instance $t_i$. In an embodiment, the bounding box is defined with six values: $x_{min}, x_{max}, Y_{min}, y_{max}, z_{min}$, and $z_{max}$, wherein:

- $x_{min}$ is calculated as the minimum value of X-axis coordinates of location of all the points constituted in $P_i$.
- $x_{max}$ is calculated as the maximum value of X-axis coordinates of location of all the points constituted in $P_i$.
- Other values are similarly calculated with $y_{min}$, $y_{max}$ pertaining to Y-axis, and $z_{min}$, $z_{max}$ pertaining to Z-axis.

At step 206b, a voxel space is created based on the bounding box defined at step 206a that is associated with the set of 2-D image slices acquired at each time-instance using the 3-D interpolation technique. The voxel space includes a plurality of voxels. Each voxel of the plurality of voxels is defined as a cube in the 3-D space with a side of a predefined length r.

In an embodiment, the plurality of voxels is obtained by dividing the associated bounding box along X-axis, Y-axis, and Z-axis. The plurality of voxels (total number of voxels) in the bounding box are $V_{nx} \times V_{ny} \times V_{nz}$, where $V_{nx}$, $V_{ny}$, and $V_{nz}$ represents the number of voxels in X, Y, and Z directions respectively. The value of each of $V_{nx}$, $V_{ny}$, and $V_{nz}$ are calculated using below equations where only the integral parts are retained.

$$V_{nx} = \frac{(x_{max} - x_{min})}{r}$$

$$V_{ny} = \frac{(y_{max} - y_{min})}{r}$$

$$V_{nz} = \frac{(z_{max} - z_{min})}{r}$$

Then, the voxel properties of each voxel of the plurality of voxels are calculated. In an embodiment, the voxel properties of each voxel include a voxel centroid location and a brightness value, wherein the voxel centroid location is calculated in 3-D as the geometrical center of the voxel. The brightness value is initially assumed as 0. The brightness value for a particular voxel is calculated by using the 3-D interpolation of the brightness values of the points present in the point cloud $P_i$. The 3-D interpolation technique such as an inverse distance weighting is employed where the input is a set of source locations, target locations and source values. The output is target values with one value calculated per target location provided as input. In the present disclosure, the set of 3-D locations of all the points in the point cloud $P_i$ ($P_{il}$) are considered as the source locations, $V_{il}$ are considered as target locations, and the set of brightness values of all the points in the point cloud source values $P_i$ ($P_{ib}$) are considered as source values. The output is provided as $V_{ib}$, where the previously assigned values of 0 are updated. At this point, all the voxels are assigned the brightness values as output from the interpolation technique.

Lastly, the voxel space associated with the one or more multi-directional 2-D slices captured at each time instance is created, using the plurality of voxels and the voxel properties of each voxel. The voxel space $V_i$ for the one or more multi-directional 2-D image slices captured at time instance $t_i$ is represented as $V_i = (V_{il}, V_{ib})$, where $V_{il}$ is the set of centroid locations of all the voxels, and $V_{ib}$ is the set of brightness levels of all the voxels, with points in the same order in both $V_{il}$ and $V_{ib}$.

Finally at step 206c, the coherent voxel volume associated with the set of 2-D image slices acquired at each time-instance is created using the voxel space associated with the set of 2-D image slices acquired at associated time-instance which is created at step 206b. FIG. 5 shows an exemplary coherent voxel volume of heart in grey-scale created from the exemplary point cloud shown in FIG. 3, in accordance with some embodiments of the present disclosure. As shown in FIG. 5, the coherent voxel volume of the heart visualized by assigning the brightness value of a particular voxel to the whole 3-D area covered by that voxel. For clarity of the heart (organ), the voxels surrounding the area have been hidden.

Generation of a single voxel-volume from only parallel slices is trivial and is the norm in current industry. These parallel slices usually cover only a part of the organ, hence generation of a voxel volume for only that part is possible. Generation of the single voxel-volume using slice information captured from all different directions provide a more holistic and accurate 3-D visualization of the organ, with information from different slices merged coherently with appropriate interpolations for a representation closer to real life structure of the organ.

At step 208 of the method 200, the one or more hardware processors 104 of the system 100 are configured to create a 3-D mesh surface for each layered component of one or more layered components associated with the anatomy. The coherent voxel volume associated with the set of 2-D image slices acquired at each time-instance that is obtained at step 206 is used to create the 3-D mesh surface for each layered component by employing an iso-surface tracing technique.

Voxel volumes can be visualized to specifically build to work with displaying voxels, offering features of hiding/showing/partially showing certain voxels based on their associated brightness value so that the medical expert can view the desired part of the imaged body part at the desired depth. But calculating structural metrics like volume/surface area of a 3-D region of interest is inaccurate in voxel domain, and entails demarcating the region in all the slices and approximating the metric, accuracy of which is affected negatively if the distance between captured parallel slices is high.

If the voxel volume is converted, along with all the details within the volume, to one or more 3-D mesh-surface structure(s), then calculation of such metrics can be performed accurately using state of the art techniques, which support mesh format. In addition, mesh objects have significantly higher compatibility with existing 3-D visualization tools such as augmented reality (AR) /virtual reality (VR) tools, gaming engines, and so on.

The brightness value associated with each of the voxels is also an approximate marker of the property of the area being represented by that voxel in the human body (e.g. flesh, bone, blood etc.). The present disclosure propose not only creating one or more meshes which are parts of just the outer visible geometric hull of the voxel-volume which is not structure-less from the inside (e.g. outer wall of an organ), but multiple meshes derived from multiple brightness values of interest which are also parts of the area inside the outer hull of the organ, e.g. the inner walls, blood etc. which may be made visible if the generated meshes are sliced.

Visualizing both outer hull and inner structures together in the mesh domain helps in significantly better analytics and diagnostics than the use of only the outer hull in the domain of mesh surface. Extracting surface meshes of inner wall, outer wall, and blood flow inside the organ, specifically the heart is of utmost importance. For these parts, neither any trained machine learning model is required for automatically segmenting the parts (which itself needs a large sized dataset for that part), nor manual annotation of all the slices is needed.

In addition to the visible outer wall, other parts of interest lying within the visible wall like inner wall, blood etc. are annotated on a set of 2-D image slices. This set of images is sourced from the 2-D slices captured by imaging tools such as MRI and CT, and the set is constructed such that all the images collectively feature at least a partial view of all the parts of interest. For example, the set may contain just one image if all the parts of interest are partially or wholly visible in that one image.

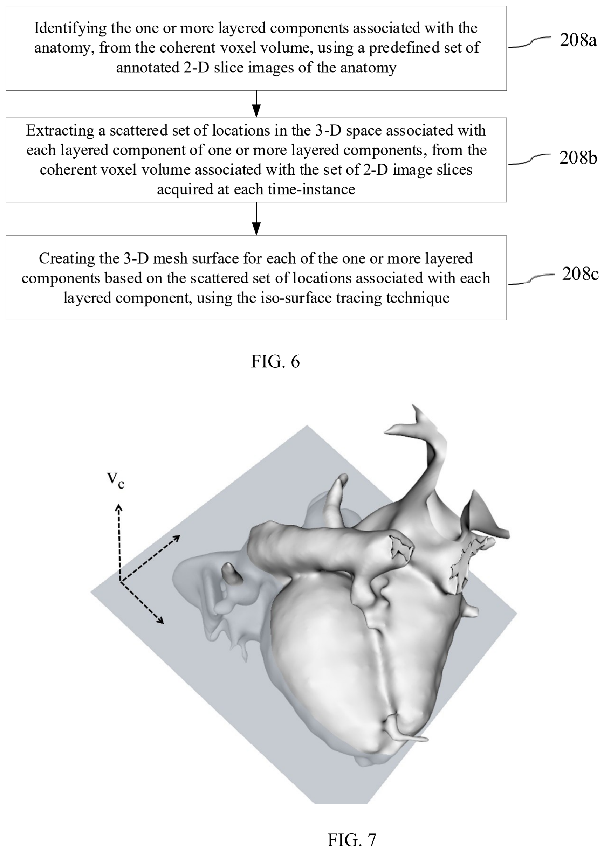

FIG. 6 is a flowchart showing the steps for creating the 3-D mesh surface for each layered component of one or more layered components associated with the anatomy, in accordance with some embodiments of the present disclosure. As shown in FIG. 6, creating the 3-D mesh surface for each layered component using the coherent voxel volume is explained through steps 208a to 208c.

At step 208a, one or more layered components associated with the anatomy are identified from the coherent voxel volume created at step 206 of the method 200, using a predefined set of annotated 2-D slice images of the anatomy. In an embodiment, the predefined set of annotated 2-D slice images of the anatomy comprises one or more annotated 2-D slice images representing one or more layered components of the anatomy.

Certain brightness values of interest of the voxels are designated as representative of certain parts/structures in the anatomy (for example, outer wall, inner wall, blood etc., of the heart) which can be inside of the visible outer hull of the organ. Such parts are identified by annotation of a few pixels or through automatic detection on a few 2-D image slices. The annotation process for obtaining the predefined set of annotated 2-D slice images is as follows:

For every part of interest, one or more pixels on the 2-D image slices is selected, where the pixel lies on a specific part of interest. This can be performed by simply clicking on the pixel using a mouse pointer and recording its brightness value using a recorder software. For example, for annotating an outer wall, a few pixels in the 2-D image slice which lie closest to the outer wall region can be chosen. The same process can be followed for all the parts of interest.

Then at step 208b, a scattered set of locations in the 3-D space associated with each layered component of one or more layered components, is extracted from the coherent voxel volume associated with the set of 2-D image slices acquired at each time-instance.

After annotating at step 208a, every part of interest can be represented as a set of brightness values of all the pixels selected for respective parts of interest. For $k^{th}$ part of interest, the collection of all brightness values can be represented as a set $B_k$. For $k^{th}$ part of interest, a single value of interest $v_k$ may be calculated using a mathematical combination of all the brightness values contained in the set $B_k$. This combination may be a statistical process like mean, median, and so on. For $k^{th}$ part of interest, a set of centroid locations of voxels $c_k$ (the scattered set of locations in the 3-D space) is defined, where each centroid location belongs to a voxel whose associated brightness value is equal to or is within a certain pre-defined tolerance of $v_k$.

Finally at step 208c, the mesh surface for each layered component is created based on the scattered set of locations associated with each layered component created at step 208b, using the iso-surface tracing technique. The iso-surface tracing technique takes input as a scattered set of locations in 3-D space ($c_k$) and provides output as a 3-D mesh constructed of triangles joined by edges such that the surface of that mesh passes through all the points in the set $c_k$. This mesh surface is a collection of triangles, which in turn are a collection of vertices and edges. The 3-D mesh surface created for $k^{th}$ part of interest can be represented as $M_k$. In another words, all the voxels in the coherent voxel volume possessing the brightness value close to the values of interest are extracted. Collection of such scattered locations of these voxels is then used to reconstruct the 3-D mesh surface.

FIG. 7 shows an exemplary 3-D mesh surface of outer hull of the heart derived from the exemplary voxel volume shown in FIG. 5, in a plane across which a cut can be made to view internal structures, in accordance with some embodiments of the present disclosure. In FIG. 7, the plane represents a medically relevant direction of 4-chamber view of the heart.

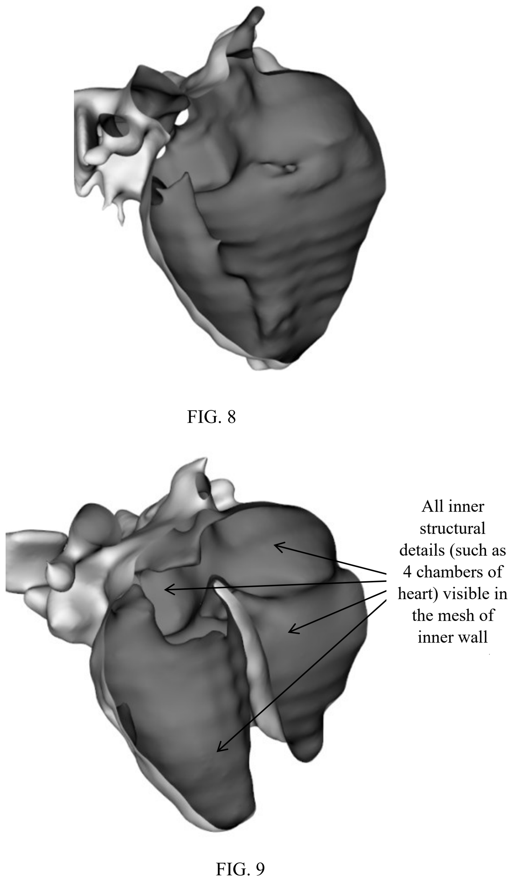

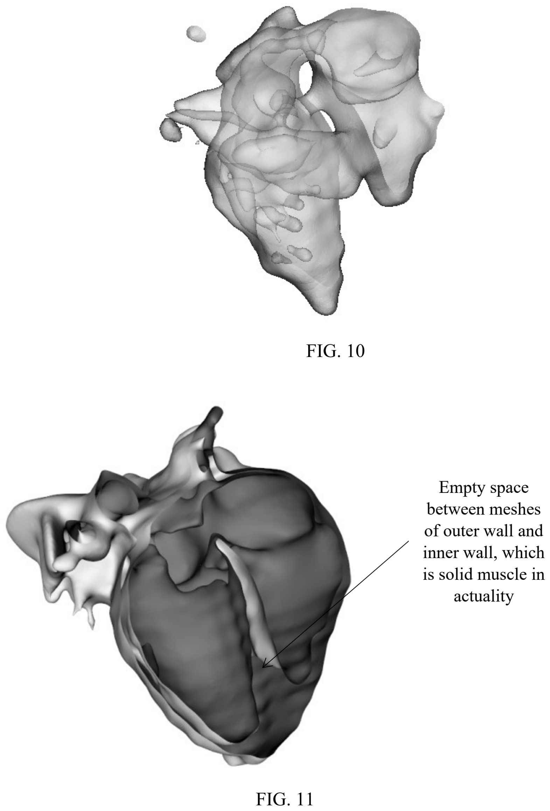

FIG. 8 shows an exemplary mesh surface of the outer wall of 3-D mesh of heart cut along the plane depicted in FIG. 7, revealing the inside portion, in accordance with some embodiments of the present disclosure. FIG. 9 shows an exemplary mesh surface of inner wall component of interest of heart cut along the plane depicted in FIG. 7, revealing significantly more details than cut view of just the outer wall depicted in depicted in FIG. 8, in accordance with some embodiments of the present disclosure. FIG. 10 shows an exemplary mesh of blood component of interest of heart cut along the plane depicted in FIG. 7, revealing detailed placement of blood in different parts of the organ, in accordance with some embodiments of the present disclosure.

At step 210 of the method 200, the one or more hardware processors 104 of the system 100 are configured to create a structurally complete sliced view of the 3-D mesh surface of the anatomy, from the 3-D mesh surface of each layered component of the one or more layered components associated with the anatomy created for each time-instance, that is created at step 208 of the method 200. A mesh manipulation and rendering technique is employed to create the structurally complete sliced view of the 3-D mesh surface of the anatomy. The structurally complete sliced view of the 3-D mesh surface of the anatomy gives the meaningful sliced view containing detailed information of the 3-D mesh surface.

When a single or multiple hulls covering the outer visible part of the anatomy is created as a mesh surface, it cannot provide a view of the structures inside the part for which the surface has been created. In order to do that, the present disclosure is proposed to cut all the meshes (slicing all the meshes) together along a certain predefined direction or directions defined by a vector $v_c$ normal to the cutting plane, which is/are medically relevant. As shown in FIG. 7, the cutting plane with the vector $v_c$ operating on the mesh $M_k$ which is represented as back of the outer structure of the heart. State of the art techniques such as the mesh manipulation and rendering technique may be implemented to cut a mesh along the plane in 3-D space. The mesh manipulation and rendering technique receives the mesh $M_k$ and the vector $v_c$ as input and provides the sliced mesh $M_{ks}$ and a closed 2-D contour $C_k$ representing closed geometry of the mesh $M_k$ lying on the cutting plane as the output.

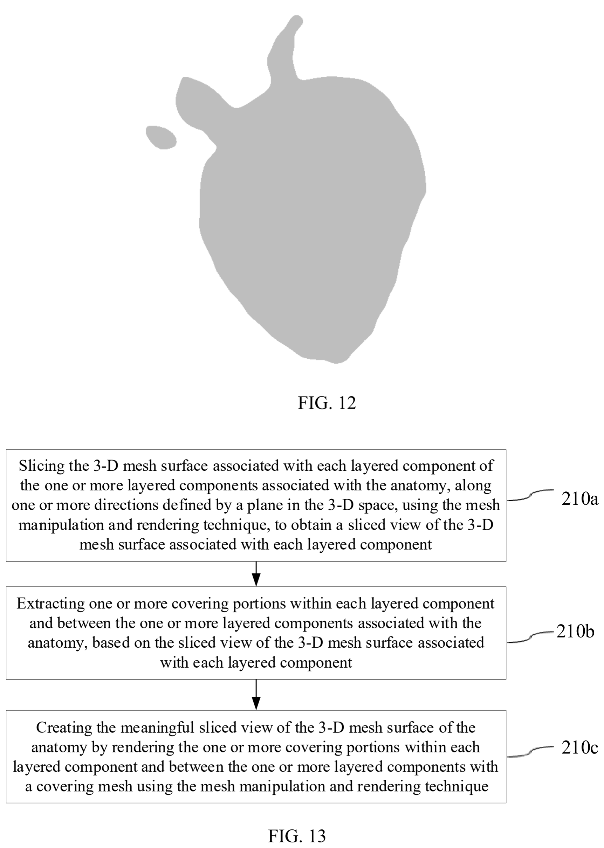

FIG. 11 shows an exemplary sliced meshes of both inner and outer walls of the heart cut along the plane as depicted FIG. 7 placed together with visible empty space between them, which is otherwise a solid area of heart muscle and fat in actuality, in accordance with some embodiments of the present disclosure. FIG. 12 shows an exemplary 2-D slice contour derived by cutting the mesh of the outer wall depicted in FIG. 8, in accordance with some embodiments of the present disclosure. Thus, except the outer shape, the cut view doesn't provide any information in the inside area at this point (figure 4), since it only reveals the reverse side of the outer shape of the hull.

In order to make the sliced view of the mesh surfaces of the anatomy meaningful, firstly, multiple structures inside of the outer visible hull are created as explained at step 208 of the method 200. Secondly, after cutting all the meshes, the empty volume in between two meshes representing inner and outer parts of solid structures (e.g. wall of the anatomy) is filled.

FIG. 13 is a flowchart showing the steps for creating a structurally complete sliced view of the 3-D mesh surface of the anatomy, in accordance with some embodiments of the present disclosure. As shown in FIG. 13, creating the structurally complete sliced view of the 3-D mesh surface of the anatomy using the 3-D mesh surface is explained through steps 210a to 210c.

At step 210a, the 3-D mesh surface associated with each layered component of the one or more layered components associated with the anatomy, is sliced along one or more directions defined by a plane in the 3-D space, using the mesh manipulation and rendering technique, to obtain a sliced view of the 3-D mesh surface associated with each layered component.

Then at step 210b, one or more covering portions within each layered component and between the one or more layered components associated with the anatomy, are extracted based on the sliced view of the 3-D mesh surface associated with each layered component. The one or more covering portions includes solid flesh, muscle, and so on of the anatomy.

Lastly at step 210c, the meaningful sliced view of the 3-D mesh surface of the anatomy is created by rendering the one or more covering portions within each layered component and between the one or more layered components with a covering mesh using the mesh manipulation and rendering technique.

As explained through steps 210a to 210c, the 3-D mesh $M_k$ is cut (sliced) with the plane with normal vector $v_c$ to yield the new sliced mesh $M_{ks}$ and the 2-D contour $C_k$. The cutting operation is performed for the 3-D meshes of all parts of interest. In an implementation, consider the parts of interest are the outer wall, the inner wall, the blood flow of the heart with respective meshes as $M_1$, $M_2$, and $M_3$. The cutting operation is performed on the meshes $M_1$, $M_2$, and $M_3$, and generates the sliced view of the meshes $M_{1s}$, $M_{2s}$, and $M_{3s}$, which are exemplified in FIG. 8, FIG. 9, and FIG. 10 respectively. It is noted that cutting only the outer visible hull does not yield any meaningful information from the inside but including inner structures in the sliced view such as $M_{2s}$ can provide a detailed inner structural information. In FIG. 9, the mesh of the inner wall the $M_{2s}$ is represented where a detailed inside view of all the 4 chambers of the heart is visible, in contrast to the mesh of the outer wall $M_{1s}$ as represented in FIG. 8, where this detailed inside view of all the 4 chambers of the heart is not visible.

It is further noted that visualizing inner and outer walls together shows an empty space between the two walls, as visible in FIG. 11. This space in the human body is a solid wall, pertaining to major part of the myocardium, which is the heart muscle. The present disclosure is proposed to cover this empty space between the outer and inner walls by a new mesh $M_{Ls}$ obtained by subtracting the slice contour meshes $C_2$ from $C_1$.

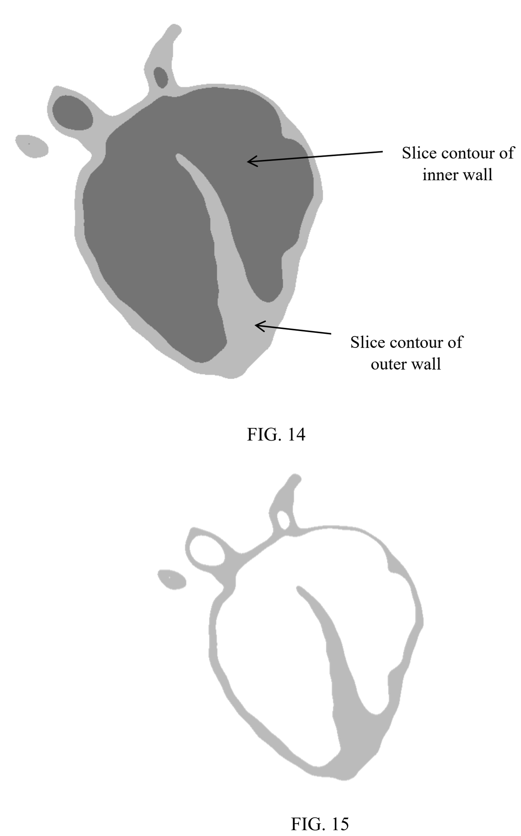

FIG. 14 shows exemplary 2-D slice contours of both the outer and inner walls derived by cutting the outer wall and the inner wall meshes depicted in FIG.8 and FIG. 9 respectively, in accordance with some embodiments of the present disclosure. As shown in FIG. 14, the 2-D slice contour of the outer wall $C_2$ as the inner darker region and the 2-D slice contour of the inner wall $C_1$ as the outer region. Only the 2-D slice contour of the inner wall $C_1$ is depicted in FIG. 12. This kind of subtraction operation can be performed using a combination of techniques such as the mesh manipulation and rendering technique. FIG. 15 shows an exemplary subtracted mesh representing one or more covering portions, in accordance with some embodiments of the present disclosure. As shown in FIG. 15, the exemplary subtracted mesh $M_{Ls}$ represents one or more covering portions.

The final meaningful (structurally complete) sliced view for a predefined direction is composed of simultaneous visualization of the meshes of all the parts of interest cut along the predefined direction defined by the vector $v_c$ normal to the cutting plane and adding the mesh $M_{Ls}$ to cover the empty parts.

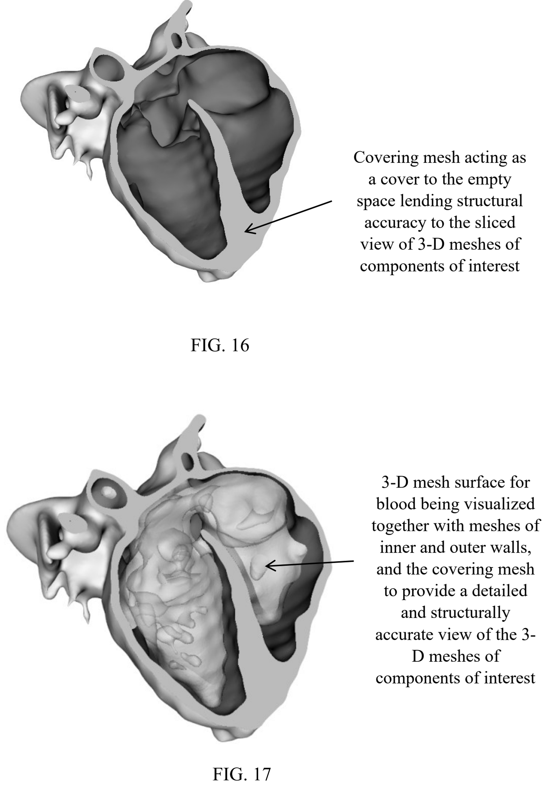

FIG. 16 shows an exemplary structurally complete sliced view where the 3-D meshes of both inner and outer walls of the heart are cut along the plane and placed together along with the covering portions depicting the solid wall, in accordance with some embodiments of the present disclosure. As shown in FIG. 16, the parts of interest such as the outer wall, the inner wall are covered by the covering portions (mesh lid $M_{Ls}$) providing a suitable view for analysis of wall-thickness, with corresponding FIG. 11 depicting the uncovered counterpart, which cannot be directly used for analytics involving wall thickness at various locations along the sliced view.

FIG. 17 shows an exemplary structurally complete sliced view where the 3-D meshes of both inner and outer walls of the heart are cut along the plane and placed together along with the covering portions depicting the solid wall and the mesh surface representing the blood flow, in accordance with some embodiments of the present disclosure. As shown in FIG. 17, the holistic sliced view of the heart is represented which includes the blood flow as an additional part of interest, all represented in the domain of mesh surfaces.

Thus, the cut/uncut 3-D meshes for one or more parts of interest may be generated for one or multiple image slices of $N_c$ captures of the operating cycle of the anatomy, as per the availability of source 2-D slice images. Then, these 3-D meshes can be visualized in succession, showing one of the $N_c$ captures at a time, which may emulate closely the operating cycle from the actuality. With inclusion of blood as part of interest in case of heart, this can be immensely helpful to emulate the blood flow in 3-D as 3-D mesh surfaces, with capability of surface area/volume metric calculation using state of the art techniques, and high compatibility with AR/VR and many other 3-D visualization tools.

Further, the cut meshes for one or more parts of interest may be generated for one or multiple directions of cutting planes. These directions for the heart may be 4 chamber, 2 chamber long axis, 2 chamber short axis, 3 chamber, and left ventricle outflow tract, along with the covering portions (mesh lid $M_{Ls}$) and blood meshes included in the final visualization.

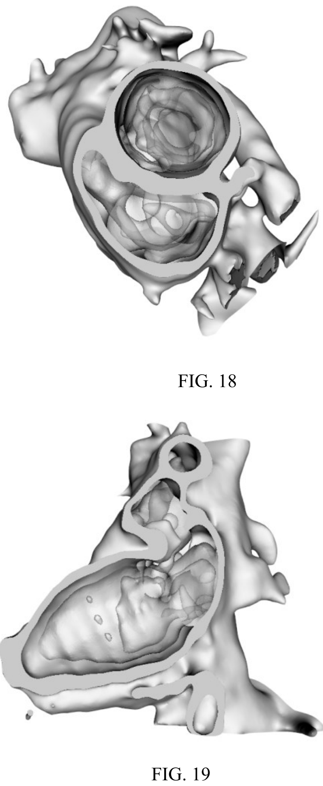

FIG. 18 shows an exemplary structurally complete sliced view with 3-D meshes of both inner and outer walls of the heart, and the 3-D mesh surface representing the blood flow cut along the plane of medically relevant 2-chamber short axis view, along with the covering portions (mesh lid $M_{Ls}$) depicting the solid wall, in accordance with some embodiments of the present disclosure. FIG. 19 shows an exemplary structurally complete sliced view with 3-D meshes of both inner and outer walls of the heart, and the 3-D mesh surface representing the blood flow cut along the plane of medically relevant 2-chamber long axis view, along with the covering portions (mesh lid $M_{Ls}$) depicting the solid wall, in accordance with some embodiments of the present disclosure.

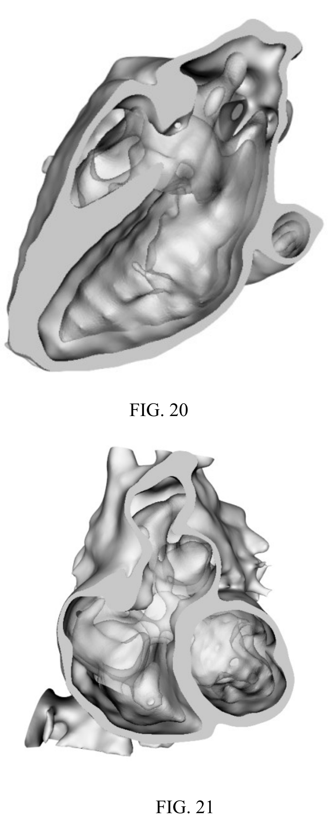

FIG. 20 shows an exemplary structurally complete sliced view with 3-D meshes of both inner and outer walls of the heart, and the 3-D mesh surface representing the blood flow cut along the plane of medically relevant 3-chamber view, along with the covering portions (mesh lid $M_{Ls}$) depicting the solid wall, in accordance with some embodiments of the present disclosure. FIG. 21 shows an exemplary structurally complete sliced view with 3-D meshes of both inner and outer walls of the heart, and the 3-D mesh surface representing the blood flow cut along the plane of medically relevant left-ventricle-outflow-tract view, along with the covering portions (mesh lid $M_{Ls}$) depicting the solid wall, in accordance with some embodiments of the present disclosure.

The methods and systems of the present disclosure discloses a solution to derive and visualize an authentic, spatially coherent full 3-D model of the anatomy derived directly from imaging techniques such as MRI and CT, from the multi-direction images slices, and not just a set of parallel slices. Such visualization can greatly assist specialists in detecting and visualizing any kind of structural, congenital disorders with a superior capability and greater coverage of the anatomy in a single 3-D structure.

The methods and systems of the present disclosure create a structurally complete (meaningful) sliced view of the internal parts along with the outer structure of the anatomy. Thus, the visualization study and the diagnosing activity can be effectively carried out for detecting abnormalities in organ walls, or other congenital defects by looking at not only the outer surface of the imaged part, but also be able to cut open the model from various directions and be able to visualize the inner details including inner walls, blood etc., together with the outer hull, as 3-D surface meshes.

The methods and systems of the present disclosure enables the single coherent volume reconstruction of full-organ from multi-direction 2-D image slices, and not just a set of parallel slices which sometimes cover just a part of the organ, leading to better analytics and diagnostics. The 3-D mesh surface reconstruction is performed for not only the external structure showing the organ's morphology, but also providing capability of viewing the internal layered details too (e.g. inner walls and blood flow visualized along with the whole model) by creating separate 3-D mesh surfaces for all layers of interest and cutting open the model from multiple directions of medical relevance. Such a capability can be highly beneficial in analyzing any kind of congenital anomaly, cardiomyopathy, valve disorders etc., which might also affect blood flow, and the same can be viewed in one single 3-D view about how this might affect other parts of the organ, which is difficult to analyze in a 2-D slice view provided directly, and also has not been available while modeling the visualization in the mesh format. This can expedite diagnostic efforts and enables a more accurate calculation of dynamic structural parameters of the organ, and thus helps with a better line of treatment.

Full and sliced reconstruction of all the organ layers is performed as 3-D mesh, which is significantly more compatible with the conventional visualization tools, involving advanced 3-D techniques like AR/VR tools, immersive gaming engines etc. Advantages of using mesh format over just voxel format for visualization:

- Voxel form of the organ's volume can be used to isolate different parts like heart walls, blood etc., but such visualizations are limited to the conventional techniques and with limited compatibilities. Approach to enable this capability by converting different parts to 3-D mesh surfaces, along with other capabilities like slicing with clear view of walls, enhances the compatibility by generating geometry models which are generically supported on any kind of 3-D visualization platform, including AR/VR platforms, making it useful not just for an effective diagnostic purpose, but also for educational purposes with immersive visualizations.
- A more significant advantage establishes in the form of possibility of calculating geometrical parameters critical to medical practitioners like surface area/volume of certain parts of the organ through time, which is not possible to perform accurately in the voxel domain, but after a reliable conversion to mesh surfaces, existing state of the arts methods can be used to readily and accurately process such metrics.

Example use case scenario:

A use case of MRI capture session was considered for the heart of a human, where the data output of 2-D image slices was provided in the form of multiple sets of parallel slices, with each set in a different slice orientation. The sets of image slices were converted into the point cloud and the point cloud information was blended to create the voxel volume with a coherent modeling of the whole structure of the heart. The 3-D mesh surface representations were derived from the voxel volume and were cut in multiple medically relevant directions to reveal the internal details of the organ while lending a meaningful view. This view was assisted by modeling not only the outer directly visible shell of the heart, but also all the layered components of interest inside the outer shell. This visualization enabled much more detail than just viewing the outer morphology of the heart in the mesh representation domain, leading to significantly more informed diagnostics.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems of 3-D reconstruction and visualization of the anatomy using parallel multi-directional 2-D image slices. According to the present disclosure, the 3-D reconstruction and visualization of the anatomy using the 2-D image slices is done in three following steps as mentioned below. In the first step, the multi-directional image information of the anatomy is combined in the form of a point cloud and converted into a voxel volume. In the second step, a 3-D mesh surface is created for each layered component associated with the anatomy from the voxel volume using an iso-surface tracing technique. In the third and last step, the layered mesh surfaces of the reconstructed anatomy are cut and opened in multiple medically relevant directions representing multiple components to represent artifacts such as covering portions (such as solid flesh/muscle), the details internal to the outer wall of the anatomy, and so on. Cutting and opening the layered mesh surfaces of the reconstructed anatomy is also non-trivial, since it requires presence of internal structure meshes in the first place.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor-implemented method (200), comprising:

   acquiring, via one or more hardware processors, a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices (202);
   generating, via the one or more hardware processors, a point cloud associated with the set of 2-D image slices acquired at each time-instance, wherein the point cloud comprises a plurality of points representing a plurality of pixels associated with the set of 2-D image slices of the anatomy acquired at each of the one or more time-instances (204);
   creating, via the one or more hardware processors, a coherent voxel volume associated with the set of 2-D image slices acquired at each time-instance, from the point cloud associated with the set of 2-D image slices, using a 3-dimensional (3-D) interpolation technique (206);
   creating, via the one or more hardware processors, a 3-D mesh surface for each layered component of one or more layered components associated with the anatomy, using (i) the coherent voxel volume associated with the set of 2-D image slices acquired at each time-instance, and (ii) an iso-surface tracing technique (208); and
   creating, via the one or more hardware processors, a structurally complete sliced view of the 3-D mesh surface of the anatomy, from the 3-D mesh surface of each layered component of the one or more layered components associated with the anatomy created for each time-instance, using a mesh manipulation and rendering technique (210).

2. The processor-implemented method (200) as claimed in claim 1, wherein the set of 2-D image slices of the anatomy is acquired in parallel from one or more predefined directions at each time-instance and at a predefined inter-slice distance in each of the one or more predefined directions.

3. The processor-implemented method (200) as claimed in claim 1, wherein each of the one or more multi-directional 2-D image slices comprises the plurality of pixels and each of the plurality of pixels comprises (i) a 3-dimensional (3-D) location with reference to a fixed coordinate system, and (ii) one or more brightness values.

4. The processor-implemented method (200) as claimed in claim 1, wherein creating the coherent voxel volume associated with the set of 2-D image slices acquired at each time-instance, comprises:

defining a bounding box in a 3-D space, using the point cloud associated with the set of 2-D image slices acquired at each time-instance (206a);
creating a voxel space based on the bounding box associated with the set of 2-D image slices acquired at each time-instance using the 3-D interpolation technique, wherein the voxel space comprises a plurality of voxels and wherein each of the plurality of voxels is defined as a cube in the 3-D space with a side of a predefined length (206b); and
creating the coherent voxel volume associated with the set of 2-D image slices acquired at each time-instance, using the voxel space associated with the set of 2-D image slices acquired at each time-instance (206c).

5. The processor-implemented method (200) as claimed in claim 1, wherein creating the 3-D mesh surface for each layered component of the one or more layered components associated with the anatomy, comprises:

identifying the one or more layered components associated with the anatomy, from the coherent voxel volume, using a predefined set of annotated 2-D slice images of the anatomy (208a);
extracting a scattered set of locations in the 3-D space associated with each layered component of one or more layered components, from the coherent voxel volume associated with the set of 2-D image slices acquired at each time-instance (208b); and
creating the 3-D mesh surface for each of the one or more layered components based on the scattered set of locations associated with each layered component, using the iso-surface tracing technique (208c).

6. The processor-implemented method (200) as claimed in claim 1, wherein creating the structurally complete sliced view of the 3-D mesh surface of the anatomy, comprises:

slicing the 3-D mesh surface associated with each layered component of the one or more layered components associated with the anatomy, along one or more directions defined by a plane in the 3-D space, using the mesh manipulation and rendering technique, to obtain a sliced view of the 3-D mesh surface associated with each layered component (210a);
extracting one or more covering portions within each layered component and between the one or more layered components associated with the anatomy, based on the sliced view of the 3-D mesh surface associated with each layered component (210b); and
creating the meaningful sliced view of the 3-D mesh surface of the anatomy by rendering the one or more covering portions within each layered component and between the one or more layered components with a covering mesh using the mesh manipulation and rendering technique (210c).

7. A system (100), comprising:

a memory (102) storing instructions;
one or more input/output (I/O) interfaces (106);
one or more hardware processors (104) coupled to the memory (102) via the one or more I/O interfaces (106), wherein the one or more hardware processors (104) are configured by the instructions to:

acquire a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices;
generate a point cloud associated with the set of 2-D image slices acquired at each time-instance, wherein the point cloud comprises a plurality of points representing a plurality of pixels associated with the set of 2-D image slices of the anatomy acquired at each of the one or more time-instances;
create a coherent voxel volume associated with the set of 2-D image slices acquired at each time-instance, from the point cloud associated with the set of 2-D image slices, using a 3-dimensional (3-D) interpolation technique;

create a 3-D mesh surface for each layered component of one or more layered components associated with the anatomy, using (i) the coherent voxel volume associated with the set of 2-D image slices acquired at each time-instance, and (ii) an iso-surface tracing technique; and

create a structurally complete sliced view of the 3-D mesh surface of the anatomy, from the 3-D mesh surface of each layered component of the one or more layered components associated with the anatomy created for each time-instance, using a mesh manipulation and rendering technique.

8. The system (100) as claimed in claim 7, wherein the one or more hardware processors (104) are configured to acquire the set of 2-D image slices of the anatomy in parallel from one or more predefined directions at each time-instance and at a predefined inter-slice distance in each of the one or more predefined directions.

9. The system (100) as claimed in claim 7, wherein each of the one or more multi-directional 2-D image slices comprises the plurality of pixels and each of the plurality of pixels comprises (i) a 3-dimensional (3-D) location with reference to a fixed coordinate system, and (ii) one or more brightness values.

10. The system (100) as claimed in claim 7, wherein the one or more hardware processors (104) are configured to create the coherent voxel volume associated with the set of 2-D image slices acquired at each time-instance, by:

defining a bounding box in a 3-D space, using the point cloud associated with the set of 2-D image slices acquired at each time-instance;

creating a voxel space based on the bounding box associated with the set of 2-D image slices acquired at each time-instance using the 3-D interpolation technique, wherein the voxel space comprises a plurality of voxels and wherein each of the plurality of voxels is defined as a cube in the 3-D space with a side of a predefined length; and

creating the coherent voxel volume associated with the set of 2-D image slices acquired at each time-instance, using the voxel space associated with the set of 2-D image slices acquired at each time-instance.

11. The system (100) as claimed in claim 7, wherein the one or more hardware processors (104) are configured to create the 3-D mesh surface for each layered component of the one or more layered components associated with the anatomy, by:

identifying the one or more layered components associated with the anatomy, from the coherent voxel volume, using a predefined set of annotated 2-D slice images of the anatomy;

extracting a scattered set of locations in the 3-D space associated with each layered component of one or more layered components, from the coherent voxel volume associated with the set of 2-D image slices acquired at each time-instance; and

creating the 3-D mesh surface for each of the one or more layered components based on the scattered set of locations associated with each layered component, using the iso-surface tracing technique.

12. The system (100) as claimed in claim 7, wherein the one or more hardware processors (104) are configured to create the structurally complete sliced view of the 3-D mesh surface of the anatomy, by:

slicing the 3-D mesh surface associated with each layered component of the one or more layered components associated with the anatomy, along one or more directions defined by a plane in the 3-D space, using the mesh manipulation and rendering technique, to obtain a sliced view of the 3-D mesh surface associated with each layered component;

extracting one or more covering portions within each layered component and between the one or more layered components associated with the anatomy, based on the sliced view of the 3-D mesh surface associated with each layered component; and

creating the meaningful sliced view of the 3-D mesh surface of the anatomy by rendering the one or more covering portions within each layered component and between the one or more layered components with a covering mesh using the mesh manipulation and rendering technique.

13. One or more non-transitory machine-readable information storage mediums comprising one or more instructions which when executed by one or more hardware processors cause:

acquiring, a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices;

generating, a point cloud associated with the set of 2-D image slices acquired at each time-instance, wherein the point cloud comprises a plurality of points representing a plurality of pixels associated with the set of 2-D image slices of the anatomy acquired at each of the one or more time-instances;

creating, a coherent voxel volume associated with the set of 2-D image slices acquired at each time-instance, from the point cloud associated with the set of 2-D image slices, using a 3-dimensional (3-D) interpolation technique;

creating, a 3-D mesh surface for each layered component of one or more layered components associated with the anatomy, using (i) the coherent voxel volume associated with the set of 2-D image slices acquired at each time-instance, and (ii) an iso-surface tracing technique; and

creating, a structurally complete sliced view of the 3-D mesh surface of the anatomy, from the 3-D mesh surface of each layered component of the one or more layered components associated with the anatomy created for each time-instance, using a mesh manipulation and rendering technique.

14. The one or more non-transitory machine readable information storage mediums as claimed in claim 13, wherein the set of 2-D image slices of the anatomy is acquired in parallel from one or more predefined directions at each time-instance and at a predefined inter-slice distance in each of the one or more predefined directions.

15. The one or more non-transitory machine readable information storage mediums as claimed in claim 13, wherein each of the one or more multi-directional 2-D image slices comprises the plurality of pixels and each of the plurality of pixels comprises (i) a 3-dimensional (3-D) location with reference to a fixed coordinate system, and (ii) one or more brightness values.

System **100**

**108**

Memory **102**

Modules **102a**

Repository **102b**

I/O interface(s) **106**

Hardware processor(s) **104**

FIG. 1

200

Acquire a set of 2-dimensional (2-D) image slices of an anatomy at each time-instance of one or more time-instances within an operating cycle of the anatomy, using one or more image acquisition techniques, wherein the set of 2-D image slices of the anatomy acquired at each time-instance comprises one or more multi-directional 2-D image slices — 202

Generate a point cloud associated with the set of 2-D image slices acquired at each time-instance, wherein the point cloud comprises a plurality of points representing a plurality of pixels associated with the set of 2-D image slices of the anatomy acquired at each of the one or more time-instances — 204

Create a coherent voxel volume associated with the set of 2-D image slices acquired at each time-instance, from the point cloud associated with the set of 2-D image slices, using a 3-dimensional (3-D) interpolation technique — 206

Create a 3-D mesh surface for each layered component of one or more layered components associated with the anatomy, using (i) the coherent voxel volume associated with the set of 2-D image slices acquired at each time-instance, and (ii) an iso-surface tracing technique — 208

Create a structurally complete sliced view of the 3-D mesh surface of the anatomy, from the 3-D mesh surface of each layered component of the one or more layered components associated with the anatomy created for each time-instance, using a mesh manipulation and rendering technique — 210

FIG. 2

FIG. 3

Defining a bounding box in a 3-D space, using the point cloud associated with the set of 2-D image slices acquired at each time-instance — 206a

Creating a voxel space based on the bounding box associated with the set of 2-D image slices acquired at each time-instance using the 3-D interpolation technique, wherein the voxel space comprises a plurality of voxels and wherein each of the plurality of voxels is defined as a cube in the 3-D space with a side of a predefined length — 206b

Creating the coherent voxel volume associated with the set of 2-D image slices acquired at each time-instance, using the voxel space associated with the set of 2-D image slices acquired at each time-instance — 206c

FIG. 4

FIG. 5

Identifying the one or more layered components associated with the anatomy, from the coherent voxel volume, using a predefined set of annotated 2-D slice images of the anatomy — 208a

Extracting a scattered set of locations in the 3-D space associated with each layered component of one or more layered components, from the coherent voxel volume associated with the set of 2-D image slices acquired at each time-instance — 208b

Creating the 3-D mesh surface for each of the one or more layered components based on the scattered set of locations associated with each layered component, using the iso-surface tracing technique — 208c

FIG. 6

$V_c$

FIG. 7

FIG. 8

All inner structural details (such as 4 chambers of heart) visible in the mesh of inner wall

FIG. 9

FIG. 10

Empty space between meshes of outer wall and inner wall, which is solid muscle in actuality

FIG. 11

FIG. 12

Slicing the 3-D mesh surface associated with each layered component of the one or more layered components associated with the anatomy, along one or more directions defined by a plane in the 3-D space, using the mesh manipulation and rendering technique, to obtain a sliced view of the 3-D mesh surface associated with each layered component — 210a

Extracting one or more covering portions within each layered component and between the one or more layered components associated with the anatomy, based on the sliced view of the 3-D mesh surface associated with each layered component — 210b

Creating the meaningful sliced view of the 3-D mesh surface of the anatomy by rendering the one or more covering portions within each layered component and between the one or more layered components with a covering mesh using the mesh manipulation and rendering technique — 210c

FIG. 13

Slice contour of
inner wall

Slice contour of
outer wall

FIG. 14

FIG. 15

Covering mesh acting as a cover to the empty space lending structural accuracy to the sliced view of 3-D meshes of components of interest

FIG. 16

3-D mesh surface for blood being visualized together with meshes of inner and outer walls, and the covering mesh to provide a detailed and structurally accurate view of the 3-D meshes of components of interest

FIG. 17

FIG. 18

FIG. 19

FIG. 20

FIG. 21

Europäisches
Patentamt

European
Patent Office

Office européen
des brevets

# EUROPEAN SEARCH REPORT

**Application Number**

EP 25 21 0069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARCEL BEETZ ET AL: "Multi-class point cloud completion networks for 3D cardiac anatomy reconstruction from cine magnetic resonance images", ARXIV.ORG, CORNELL UNIVERSITY LIBRARY, 201 OLIN LIBRARY CORNELL UNIVERSITY ITHACA, NY 14853, 17 July 2023 (2023-07-17), XP091565240, * sections 2.2,3,3.1,3.2,3.3,3.3.1,3.4; figures 1-c * | 1-15 | INV. G06T17/00 |
| X | US 10 282 846 B2 (KONINKLIJKE PHILIPS NV [NL]) 7 May 2019 (2019-05-07) column 3; * abstract * | 1-15 | |
| X | WENHUI ZHANG: "Medical Volume Reconstruction Techniques", ARXIV.ORG, CORNELL UNIVERSITY LIBRARY, 201 OLIN LIBRARY CORNELL UNIVERSITY ITHACA, NY 14853, 21 February 2018 (2018-02-21), XP081217434, * 2.1,2.2 and 3.1 * | 1-15 | |
| | | | **TECHNICAL FIELDS SEARCHED (IPC)** G06T |

The present search report has been drawn up for all claims

| Place of search | Date of completion of the search | Examiner |
|---|---|---|
| Berlin | 2 March 2026 | Kulak, Eray |

EPO FORM 1503 03.82 (P04C01)

## ANNEX TO THE EUROPEAN SEARCH REPORT
## ON EUROPEAN PATENT APPLICATION NO.

EP 25 21 0069

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2026

| Patent document cited in search report | | Publication date | Patent family member(s) | | Publication date |
|---|---|---|---|---|---|
| US 10282846 | B2 | 07-05-2019 | CN | 105814605 A | 27-07-2016 |
| | | | EP | 3080777 A1 | 19-10-2016 |
| | | | JP | 6243535 B2 | 06-12-2017 |
| | | | JP | 2017500102 A | 05-01-2017 |
| | | | US | 2016379372 A1 | 29-12-2016 |
| | | | WO | 2015086368 A1 | 18-06-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421082881